# EUROPEAN PATENT SPECIFICATION

(11) **EP 3 758 509 B1**
(45) Date of publication and mention of the grant of the patent: **25.03.2026**
(21) Application number: 19748801.8
(22) Date of filing: 30.07.2019
(51) Int. Cl.: A23L 27/30, C07D 311/30

(54) **POLYMETHOXYFLAVONES AS SWEETNESS ENHANCERS**
POLYMETHOXYFLAVON VERBINDUNGUNGEN ZUR VERSTÄRKUNG DER SÜSSIGKEIT
POLYMETHOXYFLAVONES POUR LE RENFORCEMENT DU GOUT SUCRE

(30) Priority: 01.08.2018 WO PCT/CN2018/097912
(43) Date of publication of application: 06.01.2021
(73) Proprietor: Firmenich SA, 1211 Geneva 8 (CH)
(72) Inventor: XIANG, Wen-Juan, Shanghai, 201108 (CN); YIN, Dan-Ting, Shanghai, 201108 (CN)
(74) Representative: Strych, Sebastian
(86) International application number: PCT/EP2019/070550
(87) International publication number: WO 2020/025639

(56) References cited:
- EP-A2- 0 605 261
- WO-A1-2007/083263
- CN-A- 105 475 799
- JP-A- 2009 051 738
- US-A1- 2004 022 876
- US-A1- 2004 214 882
- US-A1- 2010 015 255
- US-A1- 2015 125 557

## Description

### FIELD

Among its various aspects, the present disclosure provides compositions that comprise 3,5,6,7,3',4'-hexamethoxyflavone ("hexamethoxyquercetogetin" or "HMQ"), or 5,6,7,4'-tetramethoxyflavone ("tetra-O-methylscutellarein" or "TMS") and HMQ, and their use as sweeteners or sweetness enhancers.

### BACKGROUND

The use of non-caloric high-intensity sweeteners is increasing due to health concerns raised over childhood obesity, type II diabetes, and related illnesses. Thus, a demand exists for sweeteners having a sweetness higher than those of conventional sweeteners, such as, for example, granulated sugar (sucrose) or high-fructose corn syrup (HFCS). However, many non-caloric or low-caloric sweeteners may possess unpleasant off-flavors and/or have unexpected and less-than-desirable sweetness profiles.

Furthermore it is of interest to enhance desired flavor sensations, e.g., the sweet taste, of non- caloric or low-caloric or standard (caloric) sweeteners. Compounds that can enhance certain flavor sensations are of great interest and may allow not only improvement and/or intensification of the perceived flavor but also the ability to reach a certain flavor intensity using reduced concentrations of flavor ingredients. As one example, by employing an enhancer, less sweetener may be necessary to achieve a desired sweetness level, which may result in less calories and/or associated undesirable flavor notes/off-notes.

Thus a need exists for sweetness enhancers. Non-caloric, high intensity sweeteners are known to have sweetness levels that are significantly higher than those of conventional sweeteners. These non-caloric sweeteners often are added to the consumable products to replace at least a portion of the conventional caloric sweeteners, which results in a consumable product having a sweet flavor and a reduced amount of caloric sweetener.

In some instances, the caloric sweeteners and/or the non-caloric sweeteners are combined with other components, e.g., surfactants, emulsifiers, gums, or other sweeteners, to form sweetener compositions. These other components often improve the physical or chemical properties of the caloric or non-caloric sweetener. The sweetener compositions then may be incorporated into the respective consumable product. In most cases, the sweetener or the sweetness associated therewith is released from the consumable product quickly upon consumption of the consumable product. This release accounts for the sweetness realized by the consumer.

For some consumable products, e.g., chewing gum and other confections, once the consumable product begins to be consumed, it is desirable to have a prolonged release rate of sweetener from the consumable product. As a result, the sensation of sweetness is enjoyed by the consumer over a prolonged period of time. Conventional sweeteners, however, have been known to have more rapid release rates from the respective consumable product. As such, a majority of the sweetener is quickly released from the consumable product immediately upon consumption and very little sweetener then is left to be released during the remainder of the lifetime of the consumable product. As one example, a chewing gum comprising a conventional sweetener composition is often considered to be very sweet as the gum is initially chewed, but after some minutes of chewing, the gum is considered to be significantly less sweet.

Thus, a need exists for sweetener compositions that employ sweeteners, e.g., non-caloric, high intensity sweeteners, and that provide for prolonged release rates of these sweeteners from a consumable product to the palate of the consumer. As such, the sweetness associated with the sweetener composition may be recognized by the consumer over a longer period of time and result a prolonged sweetening sensation is realized by the consumer.

Taste-modifying compositions comprising flavone derivatives are known from, e.g., EP 0 605 261 A2.

### SUMMARY

The present invention is defined by the claims.

In a first aspect, the present disclosure provides a composition comprising hexamethoxyquercetogenin (3,5,6,7,3',4'-hexamethoxyflavone), or tetra-O-methylscutellarein and hexamethoxyquercetogenin.

According to the present claimed invention, that composition is a non-naturally occurring composition and is a powdered concentrate for use as a sweetener, a sweetening enhancer, or a flavorant. In some embodiments, the compositions, when added to a 4% w/v sucrose base at a concentration ranging from 0.001 to 20 ppm, enhances the sweetness of the 4% w/v sucrose base. In some embodiments, the composition is added to the 4% w/v sucrose base at a concentration of about 2 ppm.

In some further embodiments, the composition comprises tetra-O-methylscutellarein, and hexamethoxyquercetogetin, wherein the ratio (w/w) of tetra-O-methylscutellarein to hexamethoxyquercetogetin ranges from 1:10 to 25:1. In some further embodiments, the ratio (w/w) of tetra-O-methylscutellarein to hexamethoxyquercetogetin ranges from 1:1 to 25:1. In some further embodiments, the ratio (w/w) of tetra-O-methylscutellarein to hexamethoxyquercetogetin ranges from 5:1 to 25:1. In some further embodiments of any of the foregoing embodiments, the composition further comprises at least one additional sweetener, such as an artificial or natural sweetener.

In a second aspect, the present disclosure provides a table-top sweetener product comprising a composition according to the first aspect (including any embodiments thereof).

In a third aspect, the present disclosure provides a consumable product, selected from packaged food or beverage products, comprising the composition according to the first aspect (including any embodiments thereof).

In a fourth aspect, the present disclosure provides a use of the composition according to any of the preceding three aspects (including any embodiments thereof) to convey or enhance the sweetness of a consumable product.

In fifth aspect, the present disclosure provides a method to convey, enhance, or improve the sweetness of a consumable product, comprising: adding a composition according to any of the first three aspects (including any embodiments thereof) to the consumable product in an amount effective to convey, enhance, or improve the sweetness of the consumable product.

In a sixth aspect, the present disclosure provides a use of the composition according to any of the first three aspects (including any embodiments thereof) to convey or enhance the perception of sweetness in a subject in need thereof.

In a seventh aspect which is not part of the invention as defined by the claims, the present disclosure provides a method to convey, enhance, or improve the perception of sweetness in a subject in need thereof, wherein the method comprises contacting the subject with the composition according to any of the first three aspects (including any embodiments thereof) in an amount effective to convey, enhance, or improve the subject's perception of sweetness.

In some embodiments of any of the preceding three aspects, the amount effective of tetra-O-methylscutellarein, hexamethoxyquercetogetin, or mixtures thereof, to convey, enhance, or improve the sweetness of the consumable product ranges from 0.001 to 20 ppm. In some further such embodiments, the amount effective of tetra-O-methylscutellarein, hexamethoxyquercetogetin, or mixtures, thereof to convey, enhance, or improve the sweetness of the consumable product is about 2 ppm.

Further aspects, and embodiments thereof, are set forth below in the Detailed Description, the Drawings, the Abstract, and the Claims.

### BRIEF DESCRIPTION OF THE DRAWINGS

The following drawings are provided for purposes of illustrating various embodiments of the compositions and methods disclosed herein. The drawings are provided for illustrative purposes only, and are not intended to describe any preferred compositions or preferred methods, or to serve as a source of any limitations on the scope of the claimed inventions.

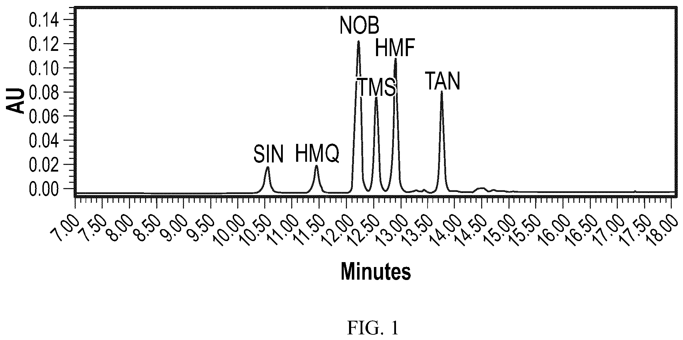

FIG. 1 shows a non-limiting example of a typical HPLC chromatogram of polymethoxyflavones extracted from a distillate residue of orange oil according to the methods described herein and described in Example 2.

### DETAILED DESCRIPTION

In the following description, reference is made to specific embodiments which may be practiced, which is shown by way of illustration.

### Polymethoxyflavones

In some aspects, the polymethoxyflavones suitable for use in the compositions disclosed herein are tetra-O-methylscutellarein and hexamethoxyquercetogetin. Such compositions include hexamethoxyquercetogenin, or tetra-O-methylscutellarein and hexamethoxyquercetogenin.

Note that, as used herein in this disclosure, the word "or" is given its broadest reasonable interpretation, and is not to be interpreted to have an either-or meaning.

As used herein, the term "tetra-O-methylscutellarein" or "TMS" refers to the compound 5,6,7,4'-tetramethoxyflavone, or the compound having the structure:

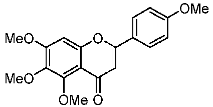

As used herein, the term "hexamethoxyquercetogetin" or "HMQ" refers to the compound 3,5,6,7,3',4'-hexamethoxyflavone, or the compound having the structure:

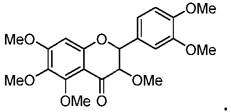

Tetra-O-methylscutellarein, hexamethoxyquercetogetin, or mixtures thereof may be obtained in any suitable way. For example, they may be isolated from biomass, or, alternatively, chemically synthesized, or obtained commercially. Non-limiting examples of biomass include fruit, leaves, stems, bark, and roots of plants.

For example, polymethoxyflavones may be found in the peel or pulp of plants from the *Citrus* genus. The particular polymethoxyflavones, and their concentration may vary, depending on the particular species of citrus plant. By way of illustration, polymethoxyflavones may be found in the peel of certain citrus species, such as sweet orange (*Citrus sinensis*), mandarin (*Citrus reticulate Blanco*) and tangerine (*Citrus tangerina*)*.* Suitable biomass includes, but is not limited to: sweet orange (*Citrus sinensis*), mandarin (*Citrus reticulata Blanco*), tangerine (*Citrus tangerina*), king orange (*Citrus nobilis*), Seville orange (*Citrus aurantium*), round kumquat (*Fortunella japonica*), ponkan (*Citrus deliciosa*), unshiu (*Citrus unshiu*), clementine (*Citrus celementina*), bitter orange (*Citrus aurantium*), grapefruit (*Citrus paradisi*), bergamot (*Citrus bergamia*), citrus hybrid such as ortanique, a hybrid between tangerine (*Citrus reticulata*) and sweet orange (*Citrus sinensis*), *Eupatorium coelestinum, Eupatorium* leucolepis, *Hedyotis corymbosa, Croton caudatus, Marrubium velutinum, Marrubium cylleneum, Conocliniopsis prasiifolia, Bauhinia guianensis, Chromolaena odorata, Praxelis clematidea, Orthosiphon stamineus, Orthosiphon stamineus,* and *Geoffroea decorticans.*

In some embodiments, the polymethoxyflavones suitable for use in the compositions provided herein are obtained from the biomass described in PCT Publication No. WO 2012/107203.

In some embodiments, the tetra-O-methylscutellarein, hexamethoxyquercetogetin, or mixtures thereof, are purified from a non-volatile residue from orange oil distillation. In some embodiments, the tetra-O-methylscutellarein, hexamethoxyquercetogetin, or mixtures thereof are purified from orange oil by first extracting orange oil in an aqueous organic solvent, and then fractionating the extract via chromatography to obtain the tetra-O-methylscutellarein, hexamethoxyquercetogetin, or a mixture thereof.

Referring to Example 1, in one non-limiting embodiment, tetra-O-methylscutellarein, hexamethoxyquercetogetin, or a mixture thereof is purified from an extract of orange oil by a method comprising the steps of:
a. dissolving the distillate residue of orange oil in cyclohexane;
b. adding an equal volume of methanol to the solution of distillate residue of orange oil in cyclohexane, and performing a liquid/liquid extraction;
c. collecting the methanol fraction containing the defatted distillate residue of orange oil obtained in step (b), and drying the collected methanol fraction;
d. adding ethyl acetate to collected methanol fraction, and loading the resulting solution to a silica gel column (flash column, 120 g, I.D. 36.6 x H 207);
e. performing a gradient elution, starting with eluting the silica gel column (120 g, I.D. 36.6 x H 207) with cyclohexane (2000 mL), and ending the elution with a 1:1 ratio of cyclohexane: ethyl acetate (1500 mL), and collecting and drying the final two fractions separately;
f. applying the collected penultimate fraction to a preparative C18(2) column, and eluting off -tetra-O-methylscutellarein and hexamethoxyquercetogetin ; and
g. applying the collected final fraction to a preparative FPF(2) column, and eluting off tetra-O-methylscutellarein.

In some further embodiments thereof, the concentration of distillate of orange oil dissolved in the cyclohexane ranges from 50 to 600 g/L, or from 100 to 500 g/L, or from 200 to 400 g/L. In some further such embodiments, the concentration of distillate of orange oil dissolved in the cyclohexane is about 300 g/L, such as dissolving 60 g of a distillate of orange oil dissolved in 200 mL of cyclohexane.

In some further embodiments, an amount of ethyl acetate is added to the concentrated methanol fraction. The v/v ratio of ethyl acetate added to the methanol concentrate, in some embodiments, ranges from 1:50 to 1:1, or from 1:25 to 1:2, or from 1:15 to 1:5. In some embodiments, about 15 mL of ethyl acetate is added to the concentrated methanol fraction.

In some embodiments, the penultimate fraction comprises tetra-O-methylscutellarein, tangeretin, hexamethoxyquercetogetin, and nobiletin. In some embodiments, the yield of the penultimate fraction of tetra-O-methylscutellarein and hexamethoxyquercetogetin ranges from 50-99%. In some embodiments, about 2.2 g of methoxyflavone products were obtained from 60 g of distillate.

In some embodiments, the final fraction comprises tetra-O-methylscutellarein and nobiletin. In some embodiments, the yield of the final fraction of tetra-O-methylscutellarein and hexamethoxyquercetogetin ranges from 50-99%. In some embodiments, about In some aspects, 1.8 g of methoxyflavone products were obtained from 60 g of distillate.

In another example, a mixture of tetra-O-methylscutellarein and hexamethoxyquercetogetin is obtained from a distillate residue of orange oil by a method comprising the steps of:
a. dissolving the distillate residue in ethanol;
b. filtering the ethanol solution, and collecting the filtered ethanol solution; and
c. concentrating the filtered ethanol solution to obtain the mixture of tetra-O-methylscutellarein and hexamethoxyquercetogetin.

In some embodiments thereof, 10 g of a distillate of orange oil is dissolved in 990 g of ethanol. In some further embodiments, the ethanol is a 50% w/w solution in water. In some other embodiments, 100 g of a distillate of orange oil is dissolved in 1900 g of ethanol. In some such embodiments, the ethanol is a 50% w/w solution in water. In some embodiments, the ethanol solution generated in step (a) is filtered through a 0.2 µm PTFE filter.

Referring to Example 2 and Figure 1, in another embodiment, tetra-O-methylscutellarein, hexamethoxyquercetogetin, or a mixture thereof, is purified from an extract of orange oil by a method comprising the steps of:
a. dissolving the distillate residue of orange oil in ethanol;
b. adding the ethanol solution on to a silica gel column;
c. performing a gradient elution, starting with eluting the silica gel column with cyclohexane, and ending the elution with a 4:1 ratio of cyclohexane: ethanol, and collecting the sub fractions;
d. applying the collected subfractions to a preparative HPLC with a reverse phase column; and
e. eluting the polymethoxyflavones off the reverse phase column.

In some embodiments thereof, 20 g of a distillate of orange oil is dissolved in 50 mL of ethanol. In some further embodiments, the ethanol is a 40% w/w solution in water. In some embodiments, the F9, F14, and F15 subfractions were collected. In some embodiments, 3,5,6,7,8,3',4'-heptamethoxyflavone and 5,6,7,8,4'-pentamethoxyflavone were purified from the F9 subfraction using an FPF(2) column. In another embodiment, tetra-O-methylscutellarein, hexamethoxyquercetogetin, and 5,6,7,8,3',4'-hexamethoxyflavone were purified from the F14 subfraction using a phenomenex C18(2) column. In another embodiment, 5,6,7,3',4'-pentamethoxyflavone were purified from the F15 subfraction using an FPF(2) column.

In some embodiments, the tetra-O-methylscutellarein, hexamethoxyquercetogetin, or a mixture thereof, are obtained according to the methods described in PCT Publication No. WO 2012/107203.

In some embodiments, the tetra-O-methylscutellarein, hexamethoxyquercetogetin, or a mixture thereof, are synthesized according to the methods described in PCT Publication No. WO 2007/083263.

In some embodiments, the tetra-O-methylscutellarein, hexamethoxyquercetogetin, or a mixture thereof, are synthesized according to the methods described in PCT Publication No. WO 2011/130705.

In some embodiments, the polymethoxyflavones suitable for use in the compositions provided herein are obtained according to the methods described in PCT Publication No. WO 2012/107203.

In some embodiments, the polymethoxyflavones suitable for use in the compositions presented herein are obtained according to the methods described in Japanese Patent Application Publication No. JP 2009051738.

In some embodiments, the polymethoxyflavones suitable for use in the compositions presented herein are obtained according to the methods described in Journal of Agricultural and Food Chemistry (1997), vol. 45(2), pp. 364-368.

In some aspects, the polymethoxyflavones suitable for use in the compositions presented herein are obtained according to the methods described in U.S. Patent Application Publication No. US20150125557A1.

In some embodiments, the polymethoxyflavones suitable for use in the compositions presented herein are obtained according to the methods described in Essenze, Derivati Agrumari (1993), vol. 63(4), pp. 395-406.

In some embodiments, the polymethoxyflavones suitable for use in the compositions presented herein are obtained according to the methods described in Molecules (2015), vol. 20, pp. 20079-20106.

In some embodiments, the polymethoxyflavones suitable for use in the compositions presented herein are obtained according to the methods described in Journal of Agricultural and Food Chemistry (2012), vol. 60(17), pp. 4336-4341.

In some embodiments, the polymethoxyflavones suitable for use in the compositions presented herein are obtained according to the methods described in Food Chemistry (2009), vol. 119(2), pp. 567-572.

In some embodiments, the polymethoxyflavones suitable for use in the compositions presented herein are obtained according to the methods described in Technologies in the Biomedical and Life Sciences (2007), vol. 846(1-2), pp. 291-297.

In some embodiments, the polymethoxyflavones suitable for use in the compositions presented herein are obtained according to the methods described in Journal of Agricultural and Food Chemistry (2006), vol. 54(12), pp. 4176-4185.

In some embodiments, the polymethoxyflavones suitable for use in the compositions presented herein are obtained according to the methods described in Biomedical Chromatography (2006), vol. 20(1), pp. 133-138.

### Compositions

There is a need for alternative (high intensity) sweeteners and sweetness enhancers which are healthy, i.e. non-caloric, non-cariogenic and ideal for diabetics, as they would also allow reducing levels of conventional caloric sweeteners and therefore provide fewer calories than an iso-sweet conventional caloric sweetener. Additionally, the need exists for sweetness enhancers that are free from off tastes, e.g. bitter or metallic tastes.

As used herein, the term "enhance" means to have an effect on a particular flavor sensation in consumables or other products placed in the oral cavity which is found more pronounced (stronger, enhanced) in its taste intensity or which is found to have an earlier onset of the flavor sensation.

As used herein, the phrase "sweetness enhancer(s)" means any compound, which is capable of enhancing or intensifying the perception of sweet taste of sweetener compositions or sweetened compositions. The phrase "sweetness enhancer" is synonymous to the terms "sweet taste potentiator," "sweetness potentiator," and sweetness intensifier." As used herein, the phrase "high intensity sweetener(s)" means any sweetener, which may in raw, extracted, purified, or any other from, singularly or in combination thereof have a sweetness potency greater than that of sucrose (common table sugar), yet have comparatively fewer calories. As used herein, the term "sweetener(s)" includes all artificial and natural sweeteners, sugar alcohols (or polyols) and sugar sweeteners (or carbohydrates).

As shown in the Examples below, the present disclosure provided the surprising and unexpected discovery that compositions comprising tetra-O-methylscutellarein (comparative), hexamethoxyquercetogetin, or a mixture thereof, are useful as a sweetener or as a sweetness enhancer (e.g., for other natural or artificial sweeteners).

Accordingly, one aspect of this disclosure provides compositions comprising hexamethoxyquercetogenin, or tetra-O-methylscutellarein and hexamethoxyquercetogenin. In some embodiments thereof, the composition, when added to a 4% w/v sucrose base at a concentration from 0.001 to 20 ppm, enhances the sweetness of the 4% w/v sucrose base.

In some embodiments, the composition, when added to a 4% w/v sucrose base at a concentration from 0.001 to 19 ppm, enhances the sweetness of the 4% w/v sucrose base. Alternatively, in some embodiments, the composition, when added to a 4% w/v sucrose base at a concentration from 0.001 to 18 ppm, enhances the sweetness of the 4% w/v sucrose base. Alternatively, in some embodiments, the composition, when added to a 4% w/v sucrose base at a concentration from 0.001 to 17 ppm, enhances the sweetness of the 4% w/v sucrose base. Alternatively, in some embodiments, the composition, when added to a 4% w/v sucrose base at a concentration from 0.001 to 16 ppm, enhances the sweetness of the 4% w/v sucrose base. Alternatively, in some embodiments, the composition, when added to a 4% w/v sucrose base at a concentration from 0.001 to 15 ppm, enhances the sweetness of the 4% w/v sucrose base. Alternatively, in some embodiments, the composition, when added to a 4% w/v sucrose base at a concentration from 0.001 to 14 ppm, enhances the sweetness of the 4% w/v sucrose base. Alternatively, in some embodiments, the composition, when added to a 4% w/v sucrose base at a concentration from 0.001 to 13 ppm, enhances the sweetness of the 4% w/v sucrose base. Alternatively, in some embodiments, the composition, when added to a 4% w/v sucrose base at a concentration from 0.001 to 12 ppm, enhances the sweetness of the 4% w/v sucrose base. Alternatively, in some embodiments, the composition, when added to a 4% w/v sucrose base at a concentration from 0.001 to 11 ppm, enhances the sweetness of the 4% w/v sucrose base. Alternatively, in some embodiments, the composition, when added to a 4% w/v sucrose base at a concentration from 0.001 to 10 ppm, enhances the sweetness of the 4% w/v sucrose base. Alternatively, in some embodiments, the composition, when added to a 4% w/v sucrose base at a concentration from 0.001 to 9 ppm, enhances the sweetness of the 4% w/v sucrose base. Alternatively, in some embodiments, the composition, when added to a 4% w/v sucrose base at a concentration from 0.001 to 8 ppm, enhances the sweetness of the 4% w/v sucrose base. Alternatively, in some embodiments, the composition, when added to a 4% w/v sucrose base at a concentration from 0.001 to 7 ppm, enhances the sweetness of the 4% w/v sucrose base. Alternatively, in some embodiments, the composition, when added to a 4% w/v sucrose base at a concentration from 0.001 to 6 ppm, enhances the sweetness of the 4% w/v sucrose base. Alternatively, in some embodiments, the composition, when added to a 4% w/v sucrose base at a concentration from 0.001 to 5 ppm, enhances the sweetness of the 4% w/v sucrose base. Alternatively, in some embodiments, the composition, when added to a 4% w/v sucrose base at a concentration from 0.001 to 4 ppm, enhances the sweetness of the 4% w/v sucrose base. Alternatively, in some embodiments, the composition, when added to a 4% w/v sucrose base at a concentration from 0.001 to 3 ppm, enhances the sweetness of the 4% w/v sucrose base. Alternatively, in some embodiments, the composition, when added to a 4% w/v sucrose base at a concentration from 0.001 to 2 ppm, enhances the sweetness of the 4% w/v sucrose base. Alternatively, in some embodiments, the composition, when added to a 4% w/v sucrose base at a concentration from 0.001 to 1 ppm, enhances the sweetness of the 4% w/v sucrose base. Alternatively, in some embodiments, the composition, when added to a 4% w/v sucrose base at a concentration from 0.001 to 0.5 ppm, enhances the sweetness of the 4% w/v sucrose base. Alternatively, in some embodiments, the composition, when added to a 4% w/v sucrose base at a concentration from 0.001 to 0.1 ppm, enhances the sweetness of the 4% w/v sucrose base. Alternatively, in some embodiments, the composition, when added to a 4% w/v sucrose base at a concentration from 0.001 to 0.05 ppm, enhances the sweetness of the 4% w/v sucrose base. Alternatively, in some embodiments, the composition, when added to a 4% w/v sucrose base at a concentration from 0.001 to 0.01 ppm, enhances the sweetness of the 4% w/v sucrose base. Alternatively, in some embodiments, the composition, when added to a 4% w/v sucrose base at a concentration from 0.001 to 0.005 ppm, enhances the sweetness of the 4% w/v sucrose base.

Alternatively, in some embodiments, the composition, when added to a 4% w/v sucrose base at a concentration from 0.005 to 20 ppm, enhances the sweetness of the 4% w/v sucrose base. Alternatively, in some embodiments, the composition, when added to a 4% w/v sucrose base at a concentration from 0.01 to 20 ppm, enhances the sweetness of the 4% w/v sucrose base. Alternatively, in some embodiments, the composition, when added to a 4% w/v sucrose base at a concentration from 0.05 to 20 ppm, enhances the sweetness of the 4% w/v sucrose base. Alternatively, in some embodiments, the composition, when added to a 4% w/v sucrose base at a concentration from 0.1 to 20 ppm, enhances the sweetness of the 4% w/v sucrose base. Alternatively, in some embodiments, the composition, when added to a 4% w/v sucrose base at a concentration from 0.5 to 20 ppm, enhances the sweetness of the 4% w/v sucrose base. Alternatively, in some embodiments, the composition, when added to a 4% w/v sucrose base at a concentration from 1 to 20 ppm, enhances the sweetness of the 4% w/v sucrose base. Alternatively, in some embodiments, the composition, when added to a 4% w/v sucrose base at a concentration from 2 to 20 ppm, enhances the sweetness of the 4% w/v sucrose base. Alternatively, in some embodiments, the composition, when added to a 4% w/v sucrose base at a concentration from 3 to 20 ppm, enhances the sweetness of the 4% w/v sucrose base. Alternatively, in some embodiments, the composition, when added to a 4% w/v sucrose base at a concentration from 4 to 20 ppm, enhances the sweetness of the 4% w/v sucrose base. Alternatively, in some embodiments, the composition, when added to a 4% w/v sucrose base at a concentration from 5 to 20 ppm, enhances the sweetness of the 4% w/v sucrose base. Alternatively, in some embodiments, the composition, when added to a 4% w/v sucrose base at a concentration from 6 to 20 ppm, enhances the sweetness of the 4% w/v sucrose base. Alternatively, in some embodiments, the composition, when added to a 4% w/v sucrose base at a concentration from 7 to 20 ppm, enhances the sweetness of the 4% w/v sucrose base. Alternatively, in some embodiments, the composition, when added to a 4% w/v sucrose base at a concentration from 8 to 20 ppm, enhances the sweetness of the 4% w/v sucrose base. Alternatively, in some embodiments, the composition, when added to a 4% w/v sucrose base at a concentration from 9 to 20 ppm, enhances the sweetness of the 4% w/v sucrose base. Alternatively, in some embodiments, the composition, when added to a 4% w/v sucrose base at a concentration from 10 to 20 ppm, enhances the sweetness of the 4% w/v sucrose base. Alternatively, in some embodiments, the composition, when added to a 4% w/v sucrose base at a concentration from 11 to 20 ppm, enhances the sweetness of the 4% w/v sucrose base. Alternatively, in some embodiments, the composition, when added to a 4% w/v sucrose base at a concentration from 12 to 20 ppm, enhances the sweetness of the 4% w/v sucrose base. Alternatively, in some embodiments, the composition, when added to a 4% w/v sucrose base at a concentration from 13 to 20 ppm, enhances the sweetness of the 4% w/v sucrose base. Alternatively, in some embodiments, the composition, when added to a 4% w/v sucrose base at a concentration from 14 to 20 ppm, enhances the sweetness of the 4% w/v sucrose base. Alternatively, in some embodiments, the composition, when added to a 4% w/v sucrose base at a concentration from 15 to 20 ppm, enhances the sweetness of the 4% w/v sucrose base. Alternatively, in some embodiments, the composition, when added to a 4% w/v sucrose base at a concentration from 16 to 20 ppm, enhances the sweetness of the 4% w/v sucrose base. Alternatively, in some embodiments, the composition, when added to a 4% w/v sucrose base at a concentration from 17 to 20 ppm, enhances the sweetness of the 4% w/v sucrose base. Alternatively, in some embodiments, the composition, when added to a 4% w/v sucrose base at a concentration from 18 to 20 ppm, enhances the sweetness of the 4% w/v sucrose base. Alternatively, in some embodiments, the composition, when added to a 4% w/v sucrose base at a concentration from 19 to 20 ppm, enhances the sweetness of the 4% w/v sucrose base.

In some embodiments, the composition, when added to a 4% w/v sucrose base at a concentration of 0.001 ppm, or 0.005 ppm, or 0.01 ppm, or 0.05 ppm, or 0.1 ppm, or 0.5 ppm, or 1 ppm, or 2 ppm, or 3 ppm, or 4 ppm, or 5 ppm, or 6 ppm, or 7 ppm, or 8 ppm, or 9 ppm, or 10 ppm, or 11 ppm, or 12 ppm, or 13 ppm, or 14 ppm, or 15 ppm, or 16 ppm, or 17 ppm, or 18 ppm, or 19 ppm, or 20 ppm, enhances the sweetness of the 4% w/v sucrose base.

In some embodiments, the composition, when added to a 4% w/v sucrose base at a concentration of 2 ppm, enhances the sweetness of the 4% w/v sucrose base.

The taste of a sample of a composition according to several aspects presented herein, with regard to sweetness and/or sweetness enhancing properties, may be assessed *in vivo* by using a panel of trained sensory evaluators experienced in the sweet taste estimation procedure, e.g. in the sensory assay described e.g. in Examples 1 and 2, using a test base comprising a 4% sucrose solution, or similar composition.

In sensory studies, panelists are asked to take a sample of the liquid to be assessed (test substance, e.g. the test base comprising a composition according to several aspects presented herein) into the mouth and after some time allowed for taste perception, to spit the sample out completely. Subsequently, the panelists are asked to rinse their mouth well with water or black tea to reduce any potential carry over effects. The tasting of a sample can be repeated if required.

Alternatively, the taste of a sample of a composition according to several aspects presented herein, with regard to sweetness or sweetness-enhancing properties, may be assessed using the methods described in PCT Publication No. WO 2012/107203.

In some embodiments, the composition comprises tetra-O-methylscutellarein, and hexamethoxyquercetogetin, wherein the ratio of tetra-O-methylscutellarein to hexamethoxyquercetogetin is from 1:10 to 25:1. In some embodiments, the ratio of tetra-O-methylscutellarein to hexamethoxyquercetogetin is from 1:1 to 25:1. In some embodiments, the ratio of tetra-O-methylscutellarein to hexamethoxyquercetogetin is from 5:1 to 25:1. In some embodiments, the ratio of tetra-O-methylscutellarein to hexamethoxyquercetogetin is 1:1. Alternatively, in some embodiments, the ratio of tetra-O-methylscutellarein to hexamethoxyquercetogetin is 2:1. Alternatively, in some embodiments, the ratio of tetra-O-methylscutellarein to hexamethoxyquercetogetin is 3:1. Alternatively, in some embodiments, the ratio of tetra-O-methylscutellarein to hexamethoxyquercetogetin is 4:1. Alternatively, in some embodiments, the ratio of tetra-O-methylscutellarein to hexamethoxyquercetogetin is 5:1. Alternatively, in some embodiments, the ratio of tetra-O-methylscutellarein to hexamethoxyquercetogetin is 6:1. Alternatively, in some embodiments, the ratio of tetra-O-methylscutellarein to hexamethoxyquercetogetin is 7:1. Alternatively, in some embodiments, the ratio of tetra-O-methylscutellarein to hexamethoxyquercetogetin is 8:1. Alternatively, in some embodiments, the ratio of tetra-O-methylscutellarein to hexamethoxyquercetogetin is 9:1. Alternatively, in some embodiments, the ratio of tetra-O-methylscutellarein to hexamethoxyquercetogetin is 10:1. Alternatively, in some embodiments, the ratio of tetra-O-methylscutellarein to hexamethoxyquercetogetin is 11:1. Alternatively, in some embodiments, the ratio of tetra-O-methylscutellarein to hexamethoxyquercetogetin is 12:1. Alternatively, in some embodiments, the ratio of tetra-O-methylscutellarein to hexamethoxyquercetogetin is 13:1. Alternatively, in some embodiments, the ratio of tetra-O-methylscutellarein to hexamethoxyquercetogetin is 14:1. Alternatively, in some embodiments, the ratio of tetra-O-methylscutellarein to hexamethoxyquercetogetin is 15:1. Alternatively, in some embodiments, the ratio of tetra-O-methylscutellarein to hexamethoxyquercetogetin is 16:1. Alternatively, in some embodiments, the ratio of tetra-O-methylscutellarein to hexamethoxyquercetogetin is 2:1. Alternatively, in some embodiments, the ratio of tetra-O-methylscutellarein to hexamethoxyquercetogetin is 17:1. Alternatively, in some embodiments, the ratio of tetra-O-methylscutellarein to hexamethoxyquercetogetin is 18:1. Alternatively, in some embodiments, the ratio of tetra-O-methylscutellarein to hexamethoxyquercetogetin is 19:1. Alternatively, in some embodiments, the ratio of tetra-O-methylscutellarein to hexamethoxyquercetogetin is 20:1. Alternatively, in some embodiments, the ratio of tetra-O-methylscutellarein to hexamethoxyquercetogetin is 21:1. Alternatively, in some embodiments, the ratio of tetra-O-methylscutellarein to hexamethoxyquercetogetin is 22:1. Alternatively, in some embodiments, the ratio of tetra-O-methylscutellarein to hexamethoxyquercetogetin is 23:1. Alternatively, in some embodiments, the ratio of tetra-O-methylscutellarein to hexamethoxyquercetogetin is 24:1. Alternatively, in some embodiments, the ratio of tetra-O-methylscutellarein to hexamethoxyquercetogetin is 25:1.

In some embodiments, the ratio of tetra-O-methylscutellarein to hexamethoxyquercetogetin is 1:2. Alternatively, in some embodiments, the ratio of tetra-O-methylscutellarein to hexamethoxyquercetogetin is 1:3. Alternatively, in some embodiments, the ratio of tetra-O-methylscutellarein to hexamethoxyquercetogetin is 1:4. Alternatively, in some embodiments, the ratio of tetra-O-methylscutellarein to hexamethoxyquercetogetin is 1:5. Alternatively, in some embodiments, the ratio of tetra-O-methylscutellarein to hexamethoxyquercetogetin is 1:6. Alternatively, in some embodiments, the ratio of tetra-O-methylscutellarein to hexamethoxyquercetogetin is 1:7. Alternatively, in some embodiments, the ratio of tetra-O-methylscutellarein to hexamethoxyquercetogetin is 1:8. Alternatively, in some embodiments, the ratio of tetra-O-methylscutellarein to hexamethoxyquercetogetin is 1:9. Alternatively, in some embodiments, the ratio of tetra-O-methylscutellarein to hexamethoxyquercetogetin is 1:10.

In some embodiments, the composition comprises the hexamethoxyquercetogenin, or the tetra-O-methylscutellarein and the hexamethoxyquercetogenin, in a purity of greater than about 60 % by weight, e.g., greater than about 70 % by weight, greater than about 80 % by weight, greater than about 90 % by weight, greater than about 98 % by weight, or greater than about 99 % by weight.

The composition may provide sweetness comparable to one to three teaspoons of granulated sugar, alternatively comparable to two teaspoons of granulated sugar. For example, the composition may contain sweetness comparable to that of granulated sugar (sucrose), and therefore can be used "spoon-for-spoon" or "cup-for-cup" in place of sugar. As used herein, the phrase "sweetness comparable" means that an experienced sensory evaluator, on average, will determine that the sweetness presented in a first composition is within a range of 80% to 120% of the sweetness presented in a second composition. The phrase "a sweetness comparable" relates to a determination ascertained by four or more experienced sensor evaluators in a sweetness matching test (designated hereinafter as "taste and spit assay"), as discussed below. Thus, for instance, 100 mg/mL of a sweetener composition according to the aspects presented herein provides "sweetness comparable" to 100 mg/mL of sucrose if the sweetener composition according to the aspects presented herein has a sweetness falling within the range of sweetness presented in 80-120 mg/mL of sucrose.

In some embodiments, the composition further comprises at least one additional sweetener. The at least one additional sweetener may be an artificial sweetener, or, alternatively, a natural sweetener.

Any suitable additional sweetener can be included in the composition. For example, in some embodiments, the at least one additional sweetener is selected from the group consisting of: abiziasaponin, abrusosides (in particular, abrusoside A, abrusoside B, abrusoside C, abrusoside D), acesulfame potassium, advantame, albiziasaponin, alitame, aspartame, superaspartame, bayunosides (in particular, bayunoside 1, bayunoside 2), brazzein, bryoside, bryonoside, bryonodulcoside, carnosifloside, carrelame, curculin, cyanin, chlorogenic acid, cyclamates and its salts, cyclocaryoside I, dihydroquercetin-3 -acetate, dihydroflavenol, dulcoside, gaudichaudioside, glycyrrhizin, glycyrrhetin acid, gypenoside, hematoxylin, isomogrosides (in particular, iso-mogroside V), lugduname, magap, mabinlins, micraculin, mogrosides (lo han guo) (in particular, mogroside IV and mogroside V), monatin and its derivatives, monellin, mukurozioside, naringin dihydrochalcone (NarDHC), neohesperidin dihydrochalcone (NHDC), neotame, osladin, pentadin, periandrin I-V, perillartine, D- phenylalanine, phlomisosides, in particular phlomisoside 1, phlomisoside 2, phlomisoside 3, phlomisoside 4, phloridzin, phyllodulcin, polpodiosides, polypodoside A, pterocaryosides, rebaudiosides (in particular rebaudioside A, rebaudioside B, rebaudioside C, rebaudioside D, rebaudioside E, rebaudioside F, rebaudioside G, rebaudioside H, rebaudioside M), rubusosides, saccharin and its salts and derivatives, scandenoside, selligueanin A, siamenosides (in particular, siamenoside I), stevia, steviolbioside, stevioside and other steviol glycosides, strogines, in particular strogin 1, strogin 2, strogin 4, suavioside A, suavioside B, suavioside G, suavioside H, suavioside I, suavioside J, sucralose, sucronate, sucrooctate, talin, telosmoside A15, thaumatin, in particular thaumatin I and II, trans-anethol, trans- cinnamaldehyde, trilobatin, D-tryptophane, erythritol, galactitol, hydrogenated starch syrups including maltitol and sorbitol syrups, inositols, isomalt, lactitol, maltitol, mannitol, xylitol, arabinose, dextrin, dextrose, fructose, high fructose corn syrup, fructooligosaccharides, fructooligosaccharide syrups, galactose, galactooligosaccharides, glucose, glucose and (hydrogenated) starch syrups/hydrolysates, isomaltulose, lactose, hydrolysed lactose, maltose, mannose, rhamnose, ribose, sucrose, tagatose, trehalose, and xylose.

In some further embodiments of any of the aforementioned embodiments, the at least one additional sweetener is selected from the sweeteners disclosed in PCT Publication No. WO 2012/107203.

Alternatively, in some other embodiments of any of the aforementioned embodiments, the at least one additional sweetener is selected from the compounds disclosed in PCT Publication No. WO 2011/130705.

Alternatively, in some other embodiments of any of the aforementioned embodiments, the at least one additional sweetener is selected from the compounds disclosed in European Patent No. 0 605 261 B1.

Alternatively, in some other embodiments of any of the aforementioned embodiments, the at least one additional sweetener is a polymethoxyflavone selected from the group consisting of: nobiletin, sinensetin, heptamethoxyflavone, and tangeretin.

Alternatively, in some other embodiments of any of the aforementioned embodiments, the at least one additional sweetener is a polymethoxyflavone disclosed in PCT Publication No. WO 2012/107203.

Alternatively, in some other embodiments of any of the aforementioned embodiments, the at least one additional sweetener is a polymethoxyflavone disclosed in PCT Publication No. WO 2011/130705.

Alternatively, in some other embodiments of any of the aforementioned embodiments, the at least one additional sweetener is a polymethoxyflavone disclosed in European Patent No. 0 605 261 B1.

As used herein, the term "nobiletin" or "NOB" refers to the compound 5,6,7,8,3',4'-hexamethoxyflavone, or the compound having the structure:

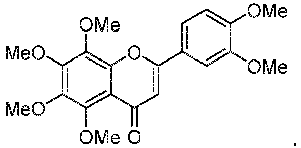

As used herein, the term "sinensetin" or "SIN" refers to the compound 5,6,7,3',4'-pentamethoxyflavone, or the compound having the structure:

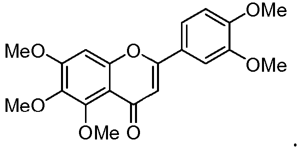

As used herein, the term "heptamethoxyflavone" or "HMF" refers to the compound 3,5,6,7,8,3',4'-heptamethoxyflavone, or the compound having the structure:

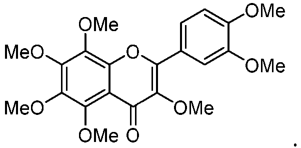

As used herein, the term "tangeretin" or "TAN" refers to the compound 5,6,7,8,4'-pentamethoxyflavone, or the compound having the structure:

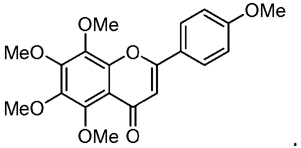

In some embodiments, the composition further comprises at least one additional sweetness enhancer, or, in some further embodiments, at least two, or at least three, additional sweetness enhancers. Suitable additional sweetness enhancers are well known in the art. In some embodiments, the at least one additional sweetness enhancer is selected from the group consisting of terpenes (such as sesquiterpenes, diterpenes, and triterpenes), flavonoids, amino acids, proteins, polyols, other known natural sweeteners (such as cinnamaldehydes, selligueains and hematoxylins), secodammarane glycosides, and analogues thereof. Exemplary sweetness enhancers include steviol glycoside such as stevioside, steviolbioside, rebaudioside A, rebaudioside B, rebaudioside C, rebaudioside D, rebaudioside E, rebaudioside F, dulcoside A, rubusoside; hernandulcin; pine rosin diterpenoid; mukurozioside; baiyunosdie; phlomisoside, such as phlomisoside I and phlomisodie II; glycyrrhizic acid; periandrins, such as periandrin I, periandrin II, periandrin III, and periandrin IV; osladin; polypodosides, such as polypodoside A and polypodoside B; mogrosides, such as mogroside IV and mogroside V; abrusoside A and abrusosdie B; cyclocariosdies, such as cyclocarioside A and cyclocarioside B; pterocaryoside A and pterocaryoside B; flavonoids, such as phyllodulcin, phloridzin, neoastilbin, and dihydroquercetin acetate; amino acids, such as glycine and monatin; proteins, such as thaumatins (thaumatin I, thaumatin II, thaumatin iii, and thaumatin IV), monellin, mabinlins (mabinlin I and mabinlin II), brazzein, miraculin, and curculin; polyols such as erythritol; cinnamaldehyde; selligueains, such as selligueain A and selligueain B; hematoxylin; and mixtures thereof. Additional exemplary sweetness enhancers include pine rosin diterpenoids; phloridizin; neoastilbin; dihydroquercetin acetate; glycine; erythritol; cinnamaldehyde; selligueain A; selligueain B; hematoxylin; rebaudioside A; rebaudioside B; rebaudioside C; rebaudioside D; rebaudioside E; dulcoside A; steviolbioside; rubusoside; stevia; stevioside; steviol 13-O-β-D-glycoside; mogroside V; Luo Han Guo; siamenoside; siamenoside I; monatin and salts thereof (monatin SS, RR, RS, SR); curculin; glycyrrhizic acid and its salts; thaumatin I; thaumatin II; thaumatin III; thaumatin IV; monellin; mabinlin I; mabinlin II; brazzein; hernandulcin; phyllodulcin; glycyphyllin; phloridzin; trilobatin; baiyunoside; osladin; polypodoside A; polypodoside B; pterocaryoside A; pterocaryoside B; mukurozioside; mukurozioside lib; phlomisoside I; phlomisoside II; periandrin I; periandrin II; periandrin III; periandrin VI; periandrin V; cyclocarioside A; cyclocarioside B; suavioside A; suavioside B; suavioside G; suavioside H; suavioside I; suavioside J; labdane glycosides; baiyunoside; gaudichaudioside A; mogroside IV; iso-mogroside; bryodulcoside; bryobioside; bryoside; bryonoside; carnosifloside V; carnosifioside VI; scandenoside R6; 1 1 -oxomogroside V; abrusoside A; abrusoside B; abrusoside C; abrusoside D; abrusoside E; gypenoside XX; glycyrrhizin; apioglycyrrhizin; araboglycyrrhizin; pentadin; perillaldehyde; rebaudioside F; steviol; 13-[(2-O-(3-O-α-D-glucopyranosyl)-β-D- glucopyranosyl-3-O-β-D-glucopyranosyl-β-D-glucopyranosyl)oxy]kaur-16-en-18-oic acid β-D-glucopyranosyl ester; 13-[(2-O-β-D-glucopyranosyl-3-O-(4-O-α-D-glucopyranosyl)- β-D-glucopyranosyl-β-D-glucopyranosyl)oxy] kaur-16-en-18-oic acid β-D- glucopyranosyl ester; 13-[(3-O-β-D-glucopyranosyl-β-D-glucopyranosyl)oxy]kaur-16-en- 18-oic acid β-D-glucopyranosyl ester; 13- hydroxy-kaur-16-en-18-oic acid β-D- glucopyranosyl ester; 13-methyl-16-oxo- 17-norkauran-18-oic acid β-D-glucopyranosyl ester; 13-[(2-O-β-D- glucopyranosyl-3-O-β-D-glucopyranosyl-β-D-glucopyranosyl)oxy] kaur-15-en-18-oic acid β-D-glucopyranosyl ester; 13-[(2-O-D-glucopyranosyl-3-O-D- glucopyranosyl-β-D-glucopyranosyl)oxy] kaur-15-en-18-oic acid; 13-[(2-O-β- D- glucopyranosyl-3-O-β-D-glucopyranosyl]-β-D-glucopyranosyl)oxy]-1 7- hydroxy-kaur-15- en-18-oic acid β-D-glucopyranosyl ester; 13-[(2-O-β-D- glucopyranosyl-3-O-D- glucopyranosyl-β-D-glucopyranosyl)oxy]-16-hydroxy kauran-18-oic acid β-D- glucopyranosyl ester; 13-[(2-O-β-D-glucopyranosyl-3-O-β-D-glucopyranosyl-β-D- glucopyranosyl)oxy]-16-hydroxy kauran-18-oic acid; isosteviol; mogroside IA; mogroside IE; mogroside 11-A; mogroside 11-E; mogroside III; mogroside V; isomogroside V; 1 1- Oxomogroside; mogrol; 1 1- oxomogrol; 1 1 -oxomogroside IA; 1-[13-hydroxykaur-16-en- 18-oate] β-D- glucopyranuronic acid; 13-[(2-O-β-D-glucopyranosyl β-D- glucopyranosyl)oxy]- 17-hydroxy-kaur-15-en-18-oic acid β-D-glucopyranosyl ester; 13-[(2-O-β-D- glucopyranosyl-β-D-glucopyranosyl)oxy] kaur-16-en-18-oic acid-(2-O-β -D-glucopyranosyl-β-D-glucopyranosyl)ester (rebaudioside E); 13-[(2-O-α-L- rhamnopyranosyl-3 -O-β-D-glucopyranosyl-β-D-glucopyranosyl)oxy] kaur- 16- en- 18-oic acid-(2-O-β-D-glucopyranosyl-β-D-glucopyranosyl) ester; 13-[(2-O- β-D-glucopyranosyl- 3-O-P-D-glucopyranosyl- -D-glucopyranosyl)oxy]- kaur-16-en-18-oic acid-(2-O-a-L-rhamnopyranosyl-β-D-glucopyranosyl) ester; 13-[(2-O-β-D-glucopyranosyl β-D-glucopyranosyl)oxy]-17-oxo-kaur-15-en-oic acid β-D-glucopyranosl ester; 13-[(2-O-(6-O- β-D-glucopyranosyl)-β-D-glucopyranosyl-β-D-glucopyranosyl)oxy] kaur-16-en-1 8-oic acid β-D-glucopyranosyl ester; 13-[(2-O-β-D-glucopyranosyl-3-O-β-D-fructofuranosyl-β- D-glucopyranosyl)oxy] kaur- 16-en-1 8-oic acid β-D-glucopyranosyl ester; 13-[(2-O-β-D-glucopyranosyl-β-D-glucopyranosyl)oxy] kaur-16-en-1 8-oic acid-(6-O-β-D- xylopyranosyl-β-D- glucopyranosyl) ester; 13-[(2-O-β-D-glucopyranosyl-β-D- glucopyranosyl)oxy] kaur-16-en-1 8-oic acid-(4-O-(2-O-α-D-glucopyranosyl)-α-D- glucopyranosyl- D-glucopyranosyl) ester; 13-[(2-O-β-D-glucopyranosyl-3-O-P-D- glucopyranosyl-β-D-glucopyranosyl)oxy] kaur-16-en-1 8-oic acid-(2-O-6- deoxy-β-D- glucopyranosyl-β-D-glucopyranosyl) ester; 13-[(2-O-β-D- glucopyranosyl-β-D- glucopyranosyl)oxy] kaur- 15-en-1 8-oic acid β-D-glucopyranosyl ester; 13-[(2-O-β-D- glucopyranosyl-3-O-β-D-xylopyranosyl-P-D-glucopyranosyl)oxy] kaur-16-en-1 8-oic acid β-D-glucopyranosyl ester; 13- [(2-O-β-D-xylopyranosyl-β-D-glucopyranosyl)oxy] kaur- 16-en-1 8-oic acid β-D- glucopyranosyl ester; 13-[(3-O-β-D-glucopyranosyl-β-D- glucopyranosyl)oxy] kaur-16-en-1 8-oic acid P-D-glucopyranosyl ester; 13-[(2-O-6-deoxy- β-D- glucopyranosyl-3-O-β-D-glucopyranosyl-β-D-glucopyranosyl)oxy] kaur-16-en- 18- oic acid β-D-glucopyranosyl ester; 13-[(2-O-6-deoxy β-D-glucopyranosyl- β-D- glucopyranosyl)oxy] kaur-16-en-1 8-oic acid β-D-glucopyranosyl ester; and mixtures thereof.

In some further embodiments, the one or more additional sweetness enhancers include rebaudioside C, rebaudioside F, rebaudioside D, 13- [(2-O-β-D-glucopyranosyl-3-O-β-D-glucopyranosyl]-β-D- glucopyranosyl)oxy]- 17-hydroxy-kaur- 15-en-1 8-oic acid β-D-glucopyranosyl ester, 13- [(2-O-(3-O-β-D-glucopyranosyl)-β-D-glucopyranosyl-3-O-β-D-glucopyranosyl-β-D- glucopyranosyl)oxy] kaur-16-en-1 8-oic acid β-D-glucopyranosyl ester, and Rubusoside. In some other embodiments, the at least one sweetness enhancer is rebaudioside A, stevioside, rebaudioside D, rebaudioside E, mogroside V, mogroside IV, brazzein, or monatin.

In some embodiments, the at least one sweetness enhancer is present in an amount at or below the sweetness detection threshold level of the at least one sweetness enhancer. In some embodiments, the at least one sweetness enhancer is present in an amount below the sweetness detection threshold level of the at least one sweetness enhancer. The sweetness detection threshold level can be specific for a particular compound. Generally, in some embodiments, the at least one sweetness enhancer is present in an amount ranging from 0.5 ppm to 1000 ppm. For example, in some further embodiments, the at least one sweetness enhancer is present in an amount ranging from 1 ppm to 300 ppm; or at least one sweetness enhancer is present in an amount ranging from 0.1 ppm to 75 ppm; or at least one sweetness enhancer is present in an amount ranging from 500 ppm to 3,000 ppm.

As used herein, the terms "sweetness threshold," "sweetness recognition threshold," and "sweetness detection threshold" are understood to mean the level at which the lowest known concentration of a certain sweet compound that is perceivable by the human sense of taste and it can vary from person to person. For example, a typical sweetness threshold level for sucrose in water can be 0.5%. Further, for example, the at least one sweetness enhancer to be used can be assayed in water at least 25% lower and at least 25% higher than the sucrose detection level of 0.5% in water to determine the sweetness threshold level. A person of skill in the art will be able to select the concentration of the at least one sweetness enhancer so that it may impart an enhanced sweetness to a composition comprising at least one sweetener. For example, a skilled artisan may select a concentration for the at least one sweetness enhancer so that the at least one sweetness enhancer does not impart any perceptible sweetness to a composition that does not comprise at least one sweetener. In some embodiments, the compounds listed above as sweeteners may also function as sweetness enhancers. Generally speaking, some sweeteners may also function as sweetness enhancers and vice versa.

### Formulations

In some aspects, the present disclosure provides formulations comprising the composition according to the foregoing aspects and embodiments thereof. In these formulations, such compositions may take any suitable form, including, but not limited to, an amorphous solid, a crystalline solid, a powder, a tablet, a liquid, a cube, a glaze, or a coating, a granulated product, an encapsulated form abound to or coated on to carriers/particles, wet or dried, or any combinations thereof. According to the present invention as claimed, the composition is a non-naturally occurring composition, which is a powdered concentrate for use as a sweetener, a sweetening enhancer, or a flavorant.

For example, in some embodiments, the formulations are provided in pre-portioned packets or ready-to-use formulations, which include a composition comprising hexamethoxyquercetogenin, or tetra-O-methylscutellarein and hexamethoxyquercetogenin. For example, in one such embodiment, a single-serving packet formulation (usually about a 1-gram portion) provides sweetness comparable to that contained in two teaspoons of granulated sugar (sucrose). It is known in the art that a "teaspoon" of sucrose contains approximately 4 grams of sucrose.

In some other embodiments, a volume of a ready-to-use formulation can provide sweetness comparable to the same volume of granulated sugar. For example, a single-serving packet of the composition (e.g., 1 gram) can provide sweetness comparable to from about 0.9 to about 9.0 grams of granulated sugar (sucrose). In another aspect, 1 gram of the composition according to several aspects presented herein contains fewer calories and carbohydrates than about 1 gram of granulated sugar, such more than 10% fewer calories, or more than 20% fewer calories, or more than 30% fewer calories, or more than 40% fewer calories, or more than 50% fewer calories, or more than 60% fewer calories, or more than 70% fewer calories, or more than 80% fewer calories, or more than 90% fewer calories.

The formulations provided herein may contain further additives known to those skilled in the art. Such additives include, but are not limited to, bubble-forming agents, bulking agents, carriers, fibers, sugar alcohols, oligo saccharides, sugars, high intensity sweeteners, nutritive sweeteners, flavorings, flavor enhancers, flavor stabilizers, acidulants, anti-caking, free-flow agents, and the like. Such additives are, for example, described in greater detail by H. Mitchell, Sweeteners and Sugar Alternatives in Food Technology (Wiley, 2006), which is incorporated herein by reference in its entirety. As used herein, the term "flavorings" includes those flavors known to the skilled person, such as natural and artificial flavors. Such flavorings may be chosen from synthetic flavor oils and flavoring aromatics or oils, oleoresins and extracts derived from plants, leaves, flowers, fruits, and so forth, and combinations thereof. Non-limiting flavor oils include spearmint oil, cinnamon oil, oil of wintergreen (methyl salicylate), peppermint oil, Japanese mint oil, clove oil, bay oil, anise oil, eucalyptus oil, thyme oil, cedar leaf oil, oil of nutmeg, allspice, oil of sage, mace, oil of bitter almonds, and cassia oil. Also useful flavorings are artificial, natural and synthetic fruit flavors such as vanilla, and citrus oils including lemon, orange, lime, grapefruit, yuzu, sudachi, and fruit essences including apple, pear, peach, grape, blueberry, strawberry, raspberry, cherry, plum, pineapple, watermelon, apricot, banana, melon, apricot, ume, cherry, raspberry, blackberry, tropical fruit, mango, mangosteen, pomegranate, papaya, and so forth. Other potential flavors include a milk flavor, a butter flavor, a cheese flavor, a cream flavor, and a yogurt flavor; a vanilla flavor; tea or coffee flavors, such as a green tea flavor, an oolong tea flavor, a tea flavor, a cocoa flavor, a chocolate flavor, and a coffee flavor; mint flavors, such as a peppermint flavor, a spearmint flavor, and a Japanese mint flavor; spicy flavors, such as an asafetida flavor, an ajowan flavor, an anise flavor, an angelica flavor, a fennel flavor, an allspice flavor, a cinnamon flavor, a camomile flavor, a mustard flavor, a cardamom flavor, a caraway flavor, a cumin flavor, a clove flavor, a pepper flavor, a coriander flavor, a sassafras flavor, a savory flavor, a Zanthoxyli Fructus flavor, a perilla flavor, a juniper berry flavor, a ginger flavor, a star anise flavor, a horseradish flavor, a thyme flavor, a tarragon flavor, a dill flavor, a capsicum flavor, a nutmeg flavor, a basil flavor, a marjoram flavor, a rosemary flavor, a bayleaf flavor, and a wasabi (Japanese horseradish) flavor; alcoholic flavors, such as a wine flavor, a whisky flavor, a brandy flavor, a rum flavor, a gin flavor, and a liqueur flavor; floral flavors; and vegetable flavors, such as an onion flavor, a garlic flavor, a cabbage flavor, a carrot flavor, a celery flavor, mushroom flavor, and a tomato flavor. These flavoring agents may be used in liquid or solid form and may be used individually or in admixture. Commonly used flavors include mints such as peppermint, menthol, spearmint, artificial vanilla, cinnamon derivatives, and various fruit flavors, whether employed individually or in admixture. Flavors may also provide breath freshening properties, particularly the mint flavors when used in combination with cooling agents.

Flavors may also provide breath-freshening properties, particularly the mint flavors when used in combination with cooling agents. These flavorings may be used in liquid or solid form and may be used individually or in admixture. Other useful flavorings include aldehydes and esters such as cinnamyl acetate, cinnamaldehyde, citral diethylacetal, dihydrocarvyl acetate, eugenyl formate, p- methylamisol, and so forth may be used. Generally any flavoring or food additive such as those described in Chemicals Used in Food Processing, publication 1274, pages 63-258 (National Academy of Sciences), may be used. This publication is incorporated herein by reference.

In some embodiments, aldehyde flavorings are also included. Further examples of aldehyde flavorings include, but are not limited to, acetaldehyde (apple), benzaldehyde (cherry, almond), anisic aldehyde (licorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavors), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2 (berry fruits), tolyl aldehyde (cherry, almond), veratraldehyde (vanilla), 2,6- dimethyl-5-heptenal, i.e., melonal (melon), 2,6-dimethyloctanal (green fruit), and 2- dodecenal (citrus, mandarin), cherry, grape, strawberry shortcake, and mixtures thereof. These listings of flavorings are merely illustrative and are not meant to limit either the term "flavoring" or the scope of the disclosure generally.

In some embodiments, the flavoring is employed in either liquid form or dried form. When employed in dried form, suitable drying means, such as spray drying the oil, may be used. Alternatively, in some other embodiments, the flavoring is absorbed onto water soluble materials, such as cellulose, starch, sugar, maltodextrin, gum arabic and so forth, and, in some further embodiments, are encapsulated. The techniques for preparing such dried forms are well-known.

In some embodiments, the flavorings are used in many distinct physical forms well-known in the art to provide an initial burst of flavor or a prolonged sensation of flavor. Without being limited thereto, such physical forms include free forms, such as spray dried, powdered, beaded forms, encapsulated forms, and mixtures thereof.

In some aspects, the present invention provides a table-top sweetener product comprising the composition according to the foregoing aspects end embodiments thereof.

The term "table-top sweetener," as used herein, refers to sweetener compositions that comprise at least one sweetener, and optionally, at least one sweetness enhancer, which can be used in the preparation of various food items or as an additive to food items. As one example, the tabletop sweetener may be used in the preparation of baked goods or other sweetened foods. As another example, the tabletop sweetener may be used to season, sweeten, or otherwise customize a prepared food item, e.g., beverages, fruit, or yoghurt.

In some embodiments, the tabletop sweetener is in a crystalline, granulated, or powder form. In some embodiments, the tabletop sweetener comprises one or more sweeteners or one or more sweetness enhancers. In one particular embodiment, the tabletop sweetener comprises one or both a caloric sweetener and a substantially non-caloric sweetener, and, in some cases, one or more sweetness enhancers. Typical examples of caloric sweeteners that may be used in tabletop sweeteners include sucrose, fructose, and glucose. Common tabletop forms of these caloric sweeteners include cane sugar, bee sugar, and the like. In recent decades, substantially non-caloric sweeteners have gained popularity. In many instances, these sweeteners can be used as substitutes for caloric sweeteners and are often referred to as "sugar substitutes."

Illustrative table-top sweetener products, which can be used are recited above, and, additionally, are disclosed in PCT Publication No. WO 2012/107203.

In some aspects, the present disclosure provides a consumable product comprising the composition according to the foregoing aspects and embodiments thereof. In some embodiments, the consumable product comprises an effective amount of a composition comprising hexamethoxyquercetogetin, or tetra-O-methylscutellarein and hexamethoxyquercetogenin. According to the invention, the consumable product which is claimed is a packaged food or beverage product.

In some embodiments, the effective amount ranges from 0.001 to 19 ppm. Alternatively, in some embodiments, the effective amount ranges from 0.001 to 18 ppm. Alternatively, in some embodiments, the effective amount ranges from 0.001 to 17 ppm. Alternatively, in some embodiments, the effective amount ranges from 0.001 to 16 ppm. Alternatively, in some embodiments, the effective amount ranges from 0.001 to 15 ppm. Alternatively, in some embodiments, the effective amount ranges from 0.001 to 14 ppm. Alternatively, in some embodiments, the effective amount ranges from 0.001 to 13 ppm. Alternatively, in some embodiments, the effective amount ranges from 0.001 to 12 ppm. Alternatively, in some embodiments, the effective amount ranges from 0.001 to 11 ppm. Alternatively, in some embodiments, the effective amount ranges from 0.001 to 10 ppm. Alternatively, in some embodiments, the effective amount ranges from 0.001 to 9 ppm. Alternatively, in some embodiments, the effective amount ranges from 0.001 to 8 ppm. Alternatively, in some embodiments, the effective amount ranges from 0.001 to 7 ppm. Alternatively, in some embodiments, the effective amount ranges from 0.001 to 6 ppm. Alternatively, in some embodiments, the effective amount ranges from 0.001 to 5 ppm. Alternatively, in some embodiments, the effective amount ranges from 0.001 to 4 ppm. Alternatively, in some embodiments, the effective amount ranges from 0.001 to 3 ppm. Alternatively, in some embodiments, the effective amount ranges from 0.001 to 2 ppm. Alternatively, in some embodiments, the effective amount ranges from 0.001 to 1 ppm. Alternatively, in some embodiments, the effective amount ranges from 0.001 to 0.5 ppm. Alternatively, in some embodiments, the effective amount ranges from 0.001 to 0.1 ppm. Alternatively, in some embodiments, the effective amount ranges from 0.001 to 0.05 ppm. Alternatively, in some embodiments, the effective amount ranges from 0.001 to 0.01 ppm. Alternatively, in some embodiments, the effective amount ranges from 0.001 to 0.005 ppm.

Alternatively, in some embodiments, the effective amount ranges from 0.005 to 20 ppm. Alternatively, in some embodiments, the effective amount ranges from 0.01 to 20 ppm. Alternatively, in some embodiments, the effective amount ranges from 0.05 to 20 ppm. Alternatively, in some embodiments, the effective amount ranges from 0.1 to 20 ppm. Alternatively, in some embodiments, the effective amount ranges from 0.5 to 20 ppm. Alternatively, in some embodiments, the effective amount ranges from 1 to 20 ppm. Alternatively, in some embodiments, the effective amount ranges from 2 to 20 ppm. Alternatively, in some embodiments, the effective amount ranges from 3 to 20 ppm. Alternatively, in some embodiments, the effective amount ranges from 4 to 20 ppm. Alternatively, in some embodiments, the effective amount ranges from 5 to 20 ppm. Alternatively, in some embodiments, the effective amount ranges from 6 to 20 ppm. Alternatively, in some embodiments, the effective amount ranges from 7 to 20 ppm. Alternatively, in some embodiments, the effective amount ranges from 8 to 20 ppm. Alternatively, in some embodiments, the effective amount ranges from 9 to 20 ppm. Alternatively, in some embodiments, the effective amount ranges from 10 to 20 ppm. Alternatively, in some embodiments, the effective amount ranges from 11 to 20 ppm. Alternatively, in some embodiments, the effective amount ranges from 12 to 20 ppm. Alternatively, in some embodiments, the effective amount ranges from 13 to 20 ppm. Alternatively, in some embodiments, the effective amount ranges from 14 to 20 ppm. Alternatively, in some embodiments, the effective amount ranges from 15 to 20 ppm. Alternatively, in some embodiments, the effective amount ranges from 16 to 20 ppm. Alternatively, in some embodiments, the effective amount ranges from 17 to 20 ppm. Alternatively, in some embodiments, the effective amount ranges from 18 to 20 ppm. Alternatively, in some embodiments, the effective amount ranges from 19 to 20 ppm.

In some embodiments, the effective amount is 0.001 ppm, or 0.005 ppm, or 0.01 ppm, or 0.05 ppm, or 0.1 ppm, or 0.5 ppm, or 1 ppm, or 2 ppm, or 3 ppm, or 4 ppm, or 5 ppm, or 6 ppm, or 7 ppm, or 8 ppm, or 9 ppm, or 10 ppm, or 11 ppm, or 12 ppm, or 13 ppm, or 14 ppm, or 15 ppm, or 16 ppm, or 17 ppm, or 18 ppm, or 19 ppm, or 20 ppm.

Consumable products include, but are not limited to, beverages, dental products, cosmetic products, pharmaceutical products and animal feed or animal food. For example, consumable products include all food products, including but not limited to cereal products, rice products, tapioca products, sago products, baker's products, biscuit products, pastry products, bread products, confectionary products, desert products, gums, chewing gums, chocolates, ices, honey products, treacle products, yeast products, baking-powder, salt and spice products, savory products, mustard products, vinegar products, sauces (condiments), tobacco products, cigars, cigarettes, processed foods, cooked fruits and vegetable products, meat and meat products, jellies, jams, fruit sauces, egg products, milk and dairy products, yoghurts, cheese products, butter and butter substitute products, milk substitute products, soy products, edible oils and fat products, medicaments, beverages, carbonated beverages, alcoholic drinks, beers, soft drinks, mineral and aerated waters and other non-alcoholic drinks, fruit drinks, fruit juices, coffee, artificial coffee, tea, cacoa, including forms requiring reconstitution, food extracts, plant extracts, meat extracts, condiments, sweeteners, nutraceuticals, gelatins, pharmaceutical and non-pharmaceutical gums, tablets, lozenges, drops, emulsions, elixirs, syrups and other preparations for making beverages, and combinations thereof.

As used herein, the term "non-alcoholic drinks" includes, but is not limited to, all nonalcoholic drinks mentioned in the Directive 2003/115/EC of 22 December 2003 and in the Directive 94/35/EC of 30 June 2004, which are incorporated herein by reference, on sweeteners for use in foodstuffs. Non-limiting examples include water-based flavored drinks, energy-reduced or with no added sugar, milk- and milk-derivative-based or fruit-juice-based drinks, energy-reduced or with no added sugar, "Gaseosa": nonalcoholic water-based drink with added carbon dioxide, sweeteners, and flavorings.

Consumable products include without limitation, water-based consumables, solid dry consumables, dairy products, dairy-derived products and dairy-alternative products. In one aspect, the consumable product is a water-based consumable product including but not limited to beverage, water, aqueous beverage, enhanced/slightly sweetened water drink, flavored carbonated and still mineral and table water, carbonated beverage, non-carbonated beverage, carbonated water, still water, soft drink, non-alcoholic drink, alcoholic drink, beer, wine, liquor, fruit drink, juice, fruit juice, vegetable juice, broth drink, coffee, tea, black tea, green tea, oolong tea, herbal infusion, cacao (e.g. water- based), tea-based drink, coffee-based drinks, cacao-based drink, infusion, syrup, frozen fruit, frozen fruit juice, water-based ice, fruit ice, sorbet, dressing, salad dressing, jams, marmalades, canned fruit, savoury, delicatessen products like delicatessen salads, sauces, ketchup, mustard, pickles and marinated fish, sauce, soup, and beverage botanical materials (e.g. whole or ground), or instant powder for reconstitution (e.g. coffee beans, ground coffee, instant coffee, cacao beans, cacao powder, instant cacao, tea leaves, instant tea powder). In another embodiment, the consumable product is a solid dry consumable product including but not limited to cereals, baked food products, biscuits, bread, breakfast cereal, cereal bar, energy bars/nutritional bars, granola, cakes, rice cakes, cookies, crackers, donuts, muffins, pastries, confectionaries, chewing gum, chocolate products, chocolate, fondant, hard candy, marshmallow, pressed tablets, snack foods, botanical materials (whole or ground), and instant powders for reconstitution.

Illustrative consumable products are also disclosed in PCT Publication No. WO 2012/107203.

### Methods and Uses

In certain aspects, the present disclosure provides a use of the composition according to the foregoing aspects and embodiments to convey, enhance, or improve the sweetness of a consumable product.

In some embodiments thereof, the present disclosure provides a method to convey, enhance, or improve the sweetness of a consumable product, comprising the steps of adding the composition according to any of the foregoing aspects and embodiments to the consumable product in an amount effective to convey, enhance, or improve the sweetness of the consumable product.

In some embodiments, the amount effective to convey, enhance, or improve the sweetness of the consumable product ranges from 0.001 to 20 ppm. Alternatively, the amount effective to convey, enhance, or improve the sweetness of the consumable product ranges from 0.001 to 19 ppm. Alternatively, the amount effective to convey, enhance, or improve the sweetness of the consumable product ranges from 0.001 to 18 ppm. Alternatively, the amount effective to convey, enhance, or improve the sweetness of the consumable product ranges from 0.001 to 17 ppm. Alternatively, the amount effective to convey, enhance, or improve the sweetness of the consumable product ranges from 0.001 to 16 ppm. Alternatively, the amount effective to convey, enhance, or improve the sweetness of the consumable product ranges from 0.001 to 15 ppm. Alternatively, the amount effective to convey, enhance, or improve the sweetness of the consumable product ranges from 0.001 to 14 ppm. Alternatively, the amount effective to convey, enhance, or improve the sweetness of the consumable product ranges from 0.001 to 13 ppm. Alternatively, the amount effective to convey, enhance, or improve the sweetness of the consumable product ranges from 0.001 to 12 ppm. Alternatively, the amount effective to convey, enhance, or improve the sweetness of the consumable product ranges from 0.001 to 11 ppm. Alternatively, the amount effective to convey, enhance, or improve the sweetness of the consumable product ranges from 0.001 to 10 ppm. Alternatively, the amount effective to convey, enhance, or improve the sweetness of the consumable product ranges from 0.001 to 9 ppm. Alternatively, the amount effective to convey, enhance, or improve the sweetness of the consumable product ranges from 0.001 to 8 ppm. Alternatively, the amount effective to convey, enhance, or improve the sweetness of the consumable product ranges from 0.001 to 7 ppm. Alternatively, the amount effective to convey, enhance, or improve the sweetness of the consumable product ranges from 0.001 to 6 ppm. Alternatively, the amount effective to convey, enhance, or improve the sweetness of the consumable product ranges from 0.001 to 5 ppm. Alternatively, the amount effective to convey, enhance, or improve the sweetness of the consumable product ranges from 0.001 to 4 ppm. Alternatively, the amount effective to convey, enhance, or improve the sweetness of the consumable product ranges from 0.001 to 3 ppm. Alternatively, the amount effective to convey, enhance, or improve the sweetness of the consumable product ranges from 0.001 to 2 ppm. Alternatively, the amount effective to convey, enhance, or improve the sweetness of the consumable product ranges from 0.001 to 1 ppm. Alternatively, the amount effective to convey, enhance, or improve the sweetness of the consumable product ranges from 0.001 to 0.5 ppm. Alternatively, the amount effective to convey, enhance, or improve the sweetness of the consumable product ranges from 0.001 to 0.01 ppm. Alternatively, the amount effective to convey, enhance, or improve the sweetness of the consumable product ranges from 0.001 to 0.005 ppm.

Alternatively, the amount effective to convey, enhance, or improve the sweetness of the consumable product ranges from 0.005 to 20 ppm. Alternatively, the amount effective to convey, enhance, or improve the sweetness of the consumable product ranges from 0.01 to 20 ppm. Alternatively, the amount effective to convey, enhance, or improve the sweetness of the consumable product ranges from 0.5 to 20 ppm. Alternatively, the amount effective to convey, enhance, or improve the sweetness of the consumable product ranges from 1 to 20 ppm. Alternatively, the amount effective to convey, enhance, or improve the sweetness of the consumable product ranges from 2 to 20 ppm. Alternatively, the amount effective to convey, enhance, or improve the sweetness of the consumable product ranges from 3 to 20 ppm. Alternatively, the amount effective to convey, enhance, or improve the sweetness of the consumable product ranges from 4 to 20 ppm. Alternatively, the amount effective to convey, enhance, or improve the sweetness of the consumable product ranges from 5 to 20 ppm. Alternatively, the amount effective to convey, enhance, or improve the sweetness of the consumable product ranges from 6 to 20 ppm. Alternatively, the amount effective to convey, enhance, or improve the sweetness of the consumable product ranges from 7 to 20 ppm. Alternatively, the amount effective to convey, enhance, or improve the sweetness of the consumable product ranges from 8 to 20 ppm. Alternatively, the amount effective to convey, enhance, or improve the sweetness of the consumable product ranges from 9 to 20 ppm. Alternatively, the amount effective to convey, enhance, or improve the sweetness of the consumable product ranges from 10 to 20 ppm. Alternatively, the amount effective to convey, enhance, or improve the sweetness of the consumable product ranges from 11 to 20 ppm. Alternatively, the amount effective to convey, enhance, or improve the sweetness of the consumable product ranges from 12 to 20 ppm. Alternatively, the amount effective to convey, enhance, or improve the sweetness of the consumable product ranges from 13 to 20 ppm. Alternatively, the amount effective to convey, enhance, or improve the sweetness of the consumable product ranges from 14 to 20 ppm. Alternatively, the amount effective to convey, enhance, or improve the sweetness of the consumable product ranges from 15 to 20 ppm. Alternatively, the amount effective to convey, enhance, or improve the sweetness of the consumable product ranges from 16 to 20 ppm. Alternatively, the amount effective to convey, enhance, or improve the sweetness of the consumable product ranges from 17 to 20 ppm. Alternatively, the amount effective to convey, enhance, or improve the sweetness of the consumable product ranges from 18 to 20 ppm. Alternatively, the amount effective to convey, enhance, or improve the sweetness of the consumable product ranges from 19 to 20 ppm.

In some embodiments, the amount effective to convey, enhance, or improve the sweetness of the consumable product is 0.001 ppm, or 0.005 ppm, or 0.01 ppm, or 0.05 ppm, or 0.1 ppm, or 0.5 ppm, or 1 ppm, or 2 ppm, or 3 ppm, or 4 ppm, or 5 ppm, or 6 ppm, or 7 ppm, or 8 ppm, or 9 ppm, or 10 ppm, or 11 ppm, or 12 ppm, or 13 ppm, or 14 ppm, or 15 ppm, or 16 ppm, or 17 ppm, or 18 ppm, or 19 ppm, or 20 ppm.

In some aspects, the amount effective to convey, enhance, or improve the sweetness of the consumable product is 2 ppm.

In some aspects, the present disclosure provides a use of the composition according to the foregoing aspects and embodiments thereof to convey, enhance, or improve the perception of sweetness in a subject in need thereof.

In some aspects, the present disclosure provides a method to convey, enhance, or improve the perception of sweetness in a subject in need thereof, comprising the steps of contacting the subject with a composition according to any of the foregoing aspects and embodiments thereof an amount effective to convey, enhance, or improve the perception of sweetness in the subject. In some embodiments, the subject is a human. In some embodiments, the contacting comprises orally administering.

In some embodiments, the amount effective to convey, enhance, or improve the perception of sweetness in the subject ranges from 0.001 to 20 ppm. Alternatively, the amount effective to convey, enhance, or improve the perception of sweetness in the subject ranges from 0.001 to 19 ppm. Alternatively, the amount effective to convey, enhance, or improve the perception of sweetness in the subject ranges from 0.001 to 18 ppm. Alternatively, the amount effective to convey, enhance, or improve the perception of sweetness in the subject ranges from 0.001 to 17 ppm. Alternatively, the amount effective to convey, enhance, or improve the perception of sweetness in the subject ranges from 0.001 to 16 ppm. Alternatively, the amount effective to convey, enhance, or improve the perception of sweetness in the subject ranges from 0.001 to 15 ppm. Alternatively, the amount effective to convey, enhance, or improve the perception of sweetness in the subject ranges from 0.001 to 14 ppm. Alternatively, the amount effective to convey, enhance, or improve the perception of sweetness in the subject ranges from 0.001 to 13 ppm. Alternatively, the amount effective to convey, enhance, or improve the perception of sweetness in the subject ranges from 0.001 to 12 ppm. Alternatively, the amount effective to convey, enhance, or improve the perception of sweetness in the subject ranges from 0.001 to 11 ppm. Alternatively, the amount effective to convey, enhance, or improve the perception of sweetness in the subject ranges from 0.001 to 10 ppm. Alternatively, the amount effective to convey, enhance, or improve the perception of sweetness in the subject ranges from 0.001 to 9 ppm. Alternatively, the amount effective to convey, enhance, or improve the perception of sweetness in the subject ranges from 0.001 to 8 ppm. Alternatively, the amount effective to convey, enhance, or improve the perception of sweetness in the subject ranges from 0.001 to 7 ppm. Alternatively, the amount effective to convey, enhance, or improve the perception of sweetness in the subject ranges from 0.001 to 6 ppm. Alternatively, the amount effective to convey, enhance, or improve the perception of sweetness in the subject ranges from 0.001 to 5 ppm. Alternatively, the amount effective to convey, enhance, or improve the perception of sweetness in the subject ranges from 0.001 to 4 ppm. Alternatively, the amount effective to convey, enhance, or improve the perception of sweetness in the subject ranges from 0.001 to 3 ppm. Alternatively, the amount effective to convey, enhance, or improve the perception of sweetness in the subject ranges from 0.001 to 2 ppm. Alternatively, the amount effective to convey, enhance, or improve the perception of sweetness in the subject ranges from 0.001 to 1 ppm. Alternatively, the amount effective to convey, enhance, or improve the perception of sweetness in the subject ranges from 0.001 to 0.5 ppm. Alternatively, the amount effective to convey, enhance, or improve the perception of sweetness in the subject ranges from 0.001 to 0.01 ppm. Alternatively, the amount effective to convey, enhance, or improve the perception of sweetness in the subject ranges from 0.001 to 0.005 ppm.

Alternatively, the amount effective to convey, enhance, or improve the perception of sweetness in the subject ranges from 0.005 to 20 ppm. Alternatively, the amount effective to convey, enhance, or improve the perception of sweetness in the subject ranges from 0.01 to 20 ppm. Alternatively, the amount effective to convey, enhance, or improve the perception of sweetness in the subject ranges from 0.5 to 20 ppm. Alternatively, the amount effective to convey, enhance, or improve the perception of sweetness in the subject ranges from 1 to 20 ppm. Alternatively, the amount effective to convey, enhance, or improve the perception of sweetness in the subject ranges from 2 to 20 ppm. Alternatively, the amount effective to convey, enhance, or improve the perception of sweetness in the subject ranges from 3 to 20 ppm. Alternatively, the amount effective to convey, enhance, or improve the perception of sweetness in the subject ranges from 4 to 20 ppm. Alternatively, the amount effective to convey, enhance, or improve the perception of sweetness in the subject ranges from 5 to 20 ppm. Alternatively, the amount effective to convey, enhance, or improve the perception of sweetness in the subject ranges from 6 to 20 ppm. Alternatively, the amount effective to convey, enhance, or improve the perception of sweetness in the subject ranges from 7 to 20 ppm. Alternatively, the amount effective to convey, enhance, or improve the perception of sweetness in the subject ranges from 8 to 20 ppm. Alternatively, the amount effective to convey, enhance, or improve the perception of sweetness in the subject ranges from 9 to 20 ppm. Alternatively, the amount effective to convey, enhance, or improve the perception of sweetness in the subject ranges from 10 to 20 ppm. Alternatively, the amount effective to convey, enhance, or improve the perception of sweetness in the subject ranges from 11 to 20 ppm. Alternatively, the amount effective to convey, enhance, or improve the perception of sweetness in the subject ranges from 12 to 20 ppm. Alternatively, the amount effective to convey, enhance, or improve the perception of sweetness in the subject ranges from 13 to 20 ppm. Alternatively, the amount effective to convey, enhance, or improve the perception of sweetness in the subject ranges from 14 to 20 ppm. Alternatively, the amount effective to convey, enhance, or improve the perception of sweetness in the subject ranges from 15 to 20 ppm. Alternatively, the amount effective to convey, enhance, or improve the perception of sweetness in the subject ranges from 16 to 20 ppm. Alternatively, the amount effective to convey, enhance, or improve the perception of sweetness in the subject ranges from 17 to 20 ppm. Alternatively, the amount effective to convey, enhance, or improve the perception of sweetness in the subject ranges from 18 to 20 ppm. Alternatively, the amount effective to convey, enhance, or improve the perception of sweetness in the subject ranges from 19 to 20 ppm.

In some aspects, the amount effective to convey, enhance, or improve the perception of sweetness in the subject is 0.001 ppm, or 0.005 ppm, or 0.01 ppm, or 0.05 ppm, or 0.1 ppm, or 0.5 ppm, or 1 ppm, or 2 ppm, or 3 ppm, or 4 ppm, or 5 ppm, or 6 ppm, or 7 ppm, or 8 ppm, or 9 ppm, or 10 ppm, or 11 ppm, or 12 ppm, or 13 ppm, or 14 ppm, or 15 ppm, or 16 ppm, or 17 ppm, or 18 ppm, or 19 ppm, or 20 ppm.

In some aspects, the amount effective to convey, enhance, or improve the perception of sweetness in the subject is 2 ppm.

The present invention is best illustrated but is not limited to the following illustrative examples.

### EXAMPLES

### Example 1: Purification of Compositions Comprising Polymethoxyflavones According to Some Aspects Presented Herein

*Purification of HMQ and TMS:* 60 g of a distillate residue of orange oil was dissolved in 200 mL cyclohexane. An equal volume of methanol was added, and a liquid-liquid extraction was performed. The methanol fraction was collected and designated "Extract 1".

Extract 1 was dissolved in 15 mL ethyl acetate and loaded on to a silica gel column. A gradient elution was then performed, beginning with cyclohexane and ending with a 1:1 ratio of cyclohexane:ethyl acetate. The last two sub-fractions "F4" and "F5" were collected and loaded on to preparative HPLC with reverse phase columns using solvent system A-water and B-acetonitrile 3,5,6,7,3',4'-hexamethoxyflavone (HMQ) and 5,6,7,4'-tetramethoxyflavone (TMS) was purified from subfraction F4 on a phenomenex preparative C18(2) (150*21.20 mm*5µm) column and 5,6,7,4'-tetramethoxyflavone (TMS) was purified from subfraction F5 on a phenomenex preparative FPF(2) column (150*21.20 mm*5µm). The identities of the purified polymethoxyflavones (5,6,7,4'-tetramethoxyflavone (TMS) and 3,5,6,7,3',4'-hexamethoxyflavone (HMQ)) were confirmed by NMR and MS spectroscopy.

*Purification of a Mixture of HMQ and TMS:* 10 g of a distillate residue of orange oil was extracted with 990 g of 50% (w/w) aqueous ethanol. The extracted solution was filtrated and concentrated under reduced pressure. The dried extract comprised both 5,6,7,4'-tetramethoxyflavone (TMS) and 3,5,6,7,3',4'-hexamethoxyflavone (HMQ) as well as other polymethoxyflavones.

### Example 2: Purification and Sensory Properties of Compositions Comprising Polymethoxyflavones According to Some Aspects Presented Herein

*Purification of Polymethoxyflavones, including HMQ, TMS (comparative), a Mixture of HMQ and*
*TMS:* 100 g of a distillate residue of orange oil was added to 1900 g of 50% (w/w) aqueous ethanol and a solid-liquid extraction was performed. The extracted solution was filtered and dried under reduced pressure. The dried extract comprised a mixture of polymethoxyflavones.

20 Grams of the dried extract was dissolved in 50 mL ethanol, and the solution was applied to a silica gel column. Solvent system comprised A: cyclohexane (2000 mL) and B: a cyclohexane/ethanol solution at a ratio of 4:1 (1500 mL) A gradient elution was performed, using a starting elution solution comprising solvent A, and concluding with solvent B. A total of 16 subfractions were obtained and subjected to UPLC-UV analysis. Subfractions F9, F14 and F15 were collected, and applied to various reverse phase preparatory HPLC columns to isolate and purify the various polymethoxyflavones.

Solvent systems A -water and B - acetonitrile were used for the peak separation by preparative HPLC with phenomenex C18(2) column (150*21.20 mm*5µm) or FPF(2) column (150*21.20 mm*5µm). 42.1 mg of 3,5,6,7,3',4'-hexamethoxyflavone (hexamethoxy quercetogetin, HMQ), 100 mg of 5,6,7,4'-tetramethoxyflavone (tetra-O-methyl scutellarein, TMS) and 152 mg of 5,6,7,8,3',4'-hexamethoxyflavone (nobiletin, NOB) were isolated from subfraction F14 using a phenomenex preparative C18(2) column; Gradient elution program (50%B to 85%B) was used for each separation. Peaks were collected using a fraction collector at UV 335 nm. 40 mg of 5,6,7,3',4'-pentamethoxyflavone (sinensetin, SIN) was isolated from subfraction F15 using a phenomenex FPF(2) column (150*21.20 mm*5µm); 220 mg of 3,5,6,7,8,3',4'-heptamethoxyflavone (HMF) and 120 mg of 5,6,7,8,4'-pentamethoxyflavone (TAN) were isolated from subfraction F9 using a FPF(2) column. Gradient elution program (55%B to 75%B) was used for each separation. Peaks were collected using a fraction collector at UV 335 nm. The structures of the isolated polymethoxyflavones were determined by NMR and MS spectroscopy, and are shown below.

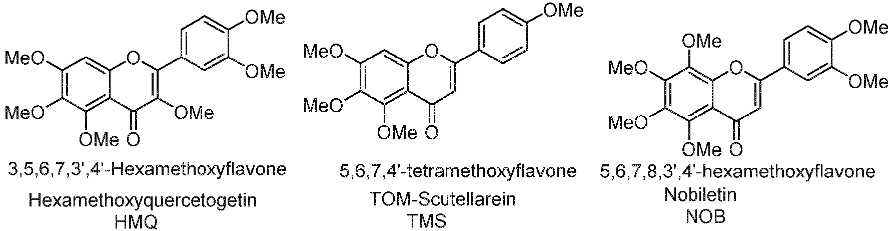

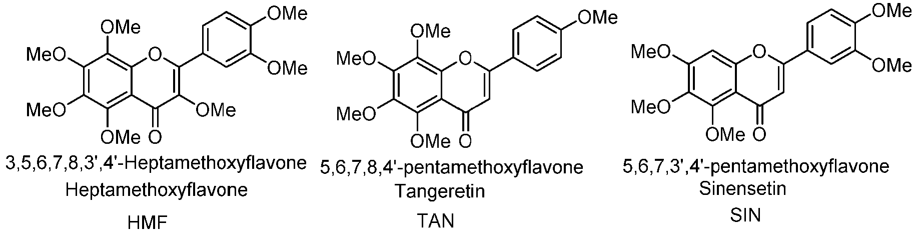

*Sensory Evaluations:* A sensory panel consisted of 25 trained panelists. The panelists were asked to evaluate the sweet taste modifying effect of the isolated individual polymethoxyflavones in a reference water solution containing 4% sucrose. The panelists evaluated samples containing TMS, HMQ, NOB, HMF, TAN and SIN (all at 5 ppm), and a mixture comprising a mixture of polymethoxyflavones (PMF mixture at 10 ppm), for taste properties (sweet) on a scale of -5 to 5 (-5 denoted strong masking effect and 5 denoted strong enhancing effect, 0 being the intensity of a reference water solution containing 4% sucrose - referred to herein as the sucrose base). The results are shown in Table 1 below.

**Table 1**

| Sucrose Base | Taste | Sample | With nose-clip (NC) | Significant level Student test (NC) |
|---|---|---|---|---|
| 4% sucrose | sweetness | 10 ppm PMF Mixture | 0.45 | ++ |
| 4% sucrose | sweetness | 5 ppm TMS | 0.55 | ++ |
| 4% sucrose | sweetness | 5 ppm HMQ | 0.57 | ++ |
| 4% sucrose | sweetness | 5 ppm NOB | 0.37 | ++ |
| 4% sucrose | sweetness | 5 ppm HMF | 0.21 | NS |
| 4% sucrose | sweetness | 5 ppm TAN | -0.30 | - |
| 4% sucrose | sweetness | 5 ppm SIN | 0.17 | NS |

| | | | | |
|---|---|---|---|---|
| Note: + 90% significant enhancement ++ 95% significant enhancement +++ 99% significant enhancement NS no significant enhancement - 90% significant negative effect | | | | |

Ten (10) ppm of the PMF Mixture was found to significantly enhance the sweetness of the sucrose base, having a confidence level of 95% with nose-clip. However, only 5 ppm TMS, 5 ppm HMQ, and 5 ppm NOB were observed to significantly enhance the sweetness of the sucrose base. Neither HMF, nor SIN had any observable enhancement of sweetness. TAN had a negative effect of sweetness.

The content of the PMF mixture was found to have all of six polymethoxyflavone (TMS, HMQ, NOB, HMF, TAN and SIN), as determined via reverse-phase HPLC, using a Luna C18(2) column and UV detector (UV at 330 nm). The HPLC chromatograph of the PMF mixture is shown in Figure 1.

*Additional Sensory Evaluations:* A sensory panel consisted of 8 trained panelists. The panelists were asked to evaluate the sweet taste modifying effect of 0.001, 0.1, 1, 2, and 20 ppm of either TMS, HMQ, or NOB in a reference water solution containing 4% sucrose, on a scale of -5 to 5 (-5 denoted strong masking effect and 5 denoted strong enhancing effect, 0 being the intensity of a reference water solution containing 4% sucrose - referred to herein as the sucrose base). The results are shown in Table 2 below.

**Table 2**

| Dosage (ppm) | TMS | HMQ | NOB |
|---|---|---|---|
| 20 | NS | NS | ++ |
| 2 | + | ++ | NS |
| 1 | ++ | NS | ++ |
| 0.1 | ++ | ++ | NS |
| 0.001 | ++ | +++ | ++ |

| | | | |
|---|---|---|---|
| Note: + 90% significant enhancement ++ 95% significant enhancement +++ 99% significant enhancement NS no significant enhancement | | | |

Both TMS and HMQ were observed to possess have better sweetness enhancement effect at doses less than or equal to 2ppm. Both TMS and HMQ possessed a significant sweetness enhancement effect at 0.1 and 0.001 ppm. In contrast, the sweetness enhancing properties of NOB were less significant.

*Additional Sensory Evaluations:* Citrus oils frequently contain more TMS than HMQ, while some may only contain TMS. The ratio of TMS to HMQ usually ranges between 2:1 and 6.5:1, the largest ratio is 25:1 [data from the book of citrus oil [edited by Giovanni Dugo and Luigi Mondello, chapter 8, the oxygen heterocyclic components of citrus essential oils].

A sensory panel consisted of 8 trained panelists. The panelists were asked to evaluate the sweet taste modifying effect of 0.5, 1, 2 ppm of either TMS or HMQ as well as 1, 1.5, 2 ppm of TMS and HMQ mixture in different ratio in a reference water solution containing 4% sucrose. The results are shown in Table 3 below.

**Table 3**

| Low sugar beverage | | | | | | | |
|---|---|---|---|---|---|---|---|
| Dosage | TMS | HMQ | Mixture TMS:HMQ (2:1) | Mixture TMS:HMQ (1:1) | Mixture TMS:HMQ (1:10) | Mixture TMS:HMQ (20:1) | Mixture TMS:HMQ (5:1) |
| 2 ppm | + | ++ | n.d. | + | + | + | ++ |
| 1.5 ppm | n.d. | n.d. | ++ | + | n.d. | n.d. | n.d. |
| 1 ppm | ++ | NS | n.d. | +++ | n.d. | n.d. | n.d. |
| 0.5ppm | NS | ++ | n.d. | n.d. | n.d. | n.d. | n.d. |

| | | | | | | | |
|---|---|---|---|---|---|---|---|
| Note: + 90% significant enhancement ++ 95% significant enhancement +++ 99% significant enhancement NS no significant enhancement n.d. No data available | | | | | | | |

A mixture of TMS and HMQ at ratio 1 to 1 was observed to possess better sweetness enhancement effect at doses 1 ppm, by comparing with 0.5 ppm of either TMS or HMQ. Similarly, a mixture of TMS and HMQ at ratio 1 to 1 was observed to possess sweetness enhancement effect at dose 2 ppm, whereas, unlike 1 ppm of TMS alone, 1 ppm of HMQ alone did not show any observable sweetness enhancement effect.

*Additional Sensory Evaluations:* A sensory panel consisted of 8 trained panelists. The panelists were asked to evaluate the sweet taste modifying effect of 0.001, 2 and 20 ppm of PMF mixture and 0.2 ppm of TMS, 0.1 ppm of HMQ, and 0.3 ppm of the mixture of TMS and HMQ at ratio of 2 to 1 in a reference low sugar fruit tea beverage solution as well as standard sugar fruit tea beverage solution. The results are shown in Table 4 below.

**Table 4**

| Reduce sugar fruit tea beverage | | | | |
|---|---|---|---|---|
| Dose | TMS | HMQ | Mixture TMS:HMQ (2:1) | PMF |
| + 20 ppm | n.d. | n.d. | n.d. | NS |
| + 2 ppm | n.d. | n.d. | n.d. | +++ |
| + 0.3ppm | n.d. | n.d. | ++ | n.d. |
| + 0.2ppm | ++ | n.d. | n.d. | n.d. |
| + 0.1ppm | n.d. | NS | n.d. | n.d. |
| Standard sugar fruit tea beverage* | ++ | ++ | ++ | ++ |

| | | | | |
|---|---|---|---|---|
| Note: + 90% significant enhancement ++ 95% significant enhancement +++ 99% significant enhancement NS no significant enhancement n.d. No data available *Standard sugar and reduced sugar fruit tea beverages have significant sweet intensity difference. | | | | |

A mixture of TMS and HMQ at ratio 2 to 1 was observed to possess a sweetness enhancement effect at 0.3 ppm, whereas 0.1 ppm of HMQ alone did not show the sweetness enhancement effect.

The PMF mixture was observed to possess a sweetness enhancement effect at doses 2 ppm.

As a conclusion, compositions containing a mixture of TMS and HMQ were observed to enhance the sweetness of reduce sugar beverage to be better than or similar with the standard sugar beverage.

## Claims

1. A composition for sweetening or sweetening enhancement, the composition comprising 3,5,6,7,3',4'-hexamethoxyflavone, or tetra-O-methylscutellarein and 3,5,6,7,3',4'-hexamethoxyflavone, wherein the composition is a non-naturally occurring composition;
wherein the composition is a powdered concentrate for use as a sweetener, a sweetening enhancer, or a flavorant.

2. The composition of claim 1, wherein the composition comprises tetra-O-methylscutellarein and 3,5,6,7,3' ,4'-hexamethoxyflavone in a weight-to-weight ratio ranging from 1:10 to 25:1.

3. The composition of claim 1 or claim 2, wherein the composition further comprises a solid carrier.

4. The composition of any one of claims 1 to 3, further comprising one or more additional sweeteners.

5. A table-top sweetener composition, which comprises a composition of any one of claims 1 to 4.

6. A packaged food or beverage product, which comprises a composition of any one of claims 1 to 4.

7. Use of a composition comprising tetra-O-methylscutellarein, 3,5,6,7,3',4'-hexamethoxyflavone, or any mixture thereof, to convey or enhance the sweetness of a consumable product by introducing the composition to the consumable product.

8. Use according to claim 7, wherein the composition is a composition according to any one of claims 1 to 4.

9. A method of conveying, enhancing, or improving the sweetness of a consumable product, comprising introducing the composition of any one of claims 1 to 4 to a consumable product.

## Patentansprüche

1. Zusammensetzung zur Süßung oder Süßungsverstärkung, wobei die Zusammensetzung 3,5,6,7,3',4'-Hexamethoxyflavon oder Tetra-O-methylscutellarein und 3,5,6,7,3',4'-Hexamethoxyflavon umfasst, wobei die Zusammensetzung eine nicht natürlich vorkommende Zusammensetzung ist; wobei die Zusammensetzung ein pulverförmiges Konzentrat zur Verwendung als Süßungsmittel, Süßungsverstärker oder Geschmacksstoff ist.

2. Zusammensetzung nach Anspruch 1, wobei die Zusammensetzung Tetra-O-methylscutellarein und 3,5,6,7,3',4'-Hexamethoxyflavon in einem Gewicht-zu-Gewicht-Verhältnis in dem Bereich von 1:10 bis 25:1 umfasst.

3. Zusammensetzung nach Anspruch 1 oder Anspruch 2, wobei die Zusammensetzung ferner einen festen Träger umfasst.

4. Zusammensetzung nach einem der Ansprüche 1 bis 3, ferner umfassend ein oder mehrere zusätzliche Süßungsmittel.

5. Tafelsüßungsmittelzusammensetzung umfassend eine Zusammensetzung nach einem der Ansprüche 1 bis 4.

6. Verpacktes Lebensmittel- oder Getränkeprodukt umfassend eine Zusammensetzung nach einem der Ansprüche 1 bis 4.

7. Verwendung einer Zusammensetzung, die Tetra-O-methylscutellarein, 3,5,6,7,3',4'-Hexamethoxyflavon oder ein beliebiges Gemisch davon umfasst, zum Vermitteln oder Verstärken der Süße eines verzehrbaren Produkts durch Einführen der Zusammensetzung in das verzehrbare Produkt.

8. Verwendung nach Anspruch 7, wobei die Zusammensetzung eine Zusammensetzung nach einem der Ansprüche 1 bis 4 ist.

9. Verfahren zum Vermitteln, Verstärken oder Verbessern der Süße eines verzehrbaren Produkts, umfassend Einführen der Zusammensetzung nach einem der Ansprüche 1 bis 4 in ein verzehrbares Produkt.

## Revendications

1. Composition pour l'édulcoration ou l'augmentation de l'édulcoration, la composition comprenant de la 3,5,6,7,3',4'-hexaméthoxyflavone, ou de la tétra-O-méthylscutellaréine et de la 3,5,6,7,3',4'-hexaméthoxyflavone, dans laquelle la composition est une composition non naturelle ;
la composition étant un concentré en poudre pour utilisation en tant qu'édulcorant, agent d'augmentation de l'édulcoration ou agent aromatisant.

2. Composition selon la revendication 1, dans laquelle la composition comprend de la tétra-O-méthylscutellaréine et de la 3,5,6,7,3',4'-hexaméthoxyflavone dans un rapport en poids-sur-poids allant de 1:10 à 25:1.

3. Composition selon la revendication 1 ou la revendication 2, dans laquelle la composition comprend en outre un véhicule solide.

4. Composition selon l'une quelconque des revendications 1 à 3, comprenant en outre un ou plusieurs édulcorants supplémentaires.

5. Composition d'édulcorant de table, qui comprend une composition selon l'une quelconque des revendications 1 à 4.

6. Produit alimentaire ou de boisson emballé, qui comprend une composition selon l'une quelconque des revendications 1 à 4.

7. Utilisation d'une composition comprenant de la tétra-O-méthylscutellaréine, de la 3,5,6,7,3',4'-hexaméthoxyflavone, ou tout mélange de celles-ci, pour véhiculer ou augmenter la sucrosité d'un produit consommable par introduction de la composition dans le produit consommable.

8. Utilisation selon la revendication 7, dans laquelle la composition est une composition selon l'une quelconque des revendications 1 à 4.

9. Procédé de transport, d'augmentation ou d'amélioration de la sucrosité d'un produit consommable, comprenant l'introduction de la composition selon l'une quelconque des revendications 1 à 4 dans un produit consommable.
